# EUROPEAN PATENT SPECIFICATION

(11) **EP 1 835 822 B1**
(45) Date of publication and mention of the grant of the patent: **29.06.2011**
(21) Application number: 06700992.8
(22) Date of filing: 09.01.2006
(51) Int. Cl.: A41D 13/12, A41D 13/00, A41D 13/11, A42B 1/04, A61B 19/04

(54) **GARMENTS, SUCH AS GOWNS, FACE MASKS, GLOVES AND HEADWEAR FOR PERSONNEL INVOLVED IN SURGICAL OPERATIONS AND THE USE OF AN ADHESIVE IN SUCH GARMENTS**
BEKLEIDUNG WIE ETWA KITTEL, GESICHTSMASKEN, HANDSCHUHE UND KOPFBEDECKUNGEN FÜR IN CHIRURGISCHE OPERATIONEN INVOLVIERTES PERSONAL UND DIE VERWENDUNG EINES KLEBSTOFFES IN SOLCHER BEKLEIDUNG
VETEMENTS TELS QUE BLOUSES, MASQUES FACIAUX, GANTS ET CHARLOTTES DESTINES AU PERSONNEL INTERVENANT DANS DES OPERATIONS CHIRURGICALES ET UTILISATIONS DE CES VETEMENTS

(30) Priority: 11.01.2005 SE 0500060
(43) Date of publication of application: 26.09.2007
(73) Proprietor: Mölnlycke Health Care AB, 402 52 Göteborg (SE)
(72) Inventor: JOHANSSON, Helena, S-411 19 Göteborg (SE); WALLEFORS, Fredrik, S-416 69 Göteborg (SE); ERICSON, Ann-Catherine, S-436 39 Askim (SE); ERIKSON, Karl, S-44834 Floda (SE)
(74) Representative: Hyltner, Jan-Olof
(86) International application number: PCT/SE2006/000022
(87) International publication number: WO 2006/075947

(56) References cited:
- EP-A2- 0 906 730
- WO-A1-03/079920
- WO-A1-03/079920
- GB-A- 763 721
- GB-A- 2 245 148
- US-A- 5 033 115
- US-A- 5 797 146
- US-A1- 2003 136 410
- US-A1- 2004 055 078
- US-A1- 2004 055 078
- US-A1- 2004 097 798
- US-B1- 6 177 482
- DATABASE WPI Week 200276, Derwent Publications Ltd., London, GB; Class P21, AN 2002-701868, XP008114306 & JP 2002 249917 A (ISHII H) 06 September 2002

## Description

### TECHNICAL FIELD

The present invention relates to a garment of a disposable kind, such as gowns, face masks, gloves and headwear for health care personnel. The garment may be sterile or non-sterile.

### BACKGROUND ART

The personnel involved in surgical operations wear sterile garments. The garment has two functions; on the one hand, it must prevent microorganisms from the surgical personnel from reaching the operation wound and, on the other hand, it must prevent the surgical personnel from becoming contaminated and possibly infected by the patient.

The risk of complications is present in every operation. The majority of post-operative infections in surgical wounds occur during that period of the operation when it is possible for microorganisms to reach the open wound. The source of microorganisms is either exogenous, that is to say from surgical personnel and instruments, or endogenous from the patient on whom the operation is being performed.

In a clean operation, that is to say when the operation is performed in sterile tissue, the skin of the surgical personnel and the patient is the most important source of microorganisms. In operations that are susceptible to infection, such as orthopaedic surgery, normal microbiological skin contains a flora of microorganisms that can cause an infection in the operation wound.

In order to avoid the spread of bacteria from the surgical personnel to the operation wound, only materials that are impermeable to air, fluids and, in some cases, viruses, shall be used.

Parts of surgical garments are difficult to seal, however, for example around necklines and around the edge of surgical headwear. These areas are often in contact with and chafe against the skin, with the associated increased risk of skin particles becoming detached and then contaminating the patient's operation wound. A healthy person can shed thousands of skin particles, which carry bacteria on them, every minute when moving around.

The skin beneath the surgical gown, the face mask and the surgical headwear is heated up, and this makes it easier for air-borne particles to find their way up from the non-sterile area under through the gap between the garment and the skin and to constitute a potential risk of contamination of the operation wound.

One object of the present invention is to reduce the risk of fluid-borne or air-borne bacteria gaining entry via openings.

A further object is to prevent condensation on spectacles and to reduce the risk of mechanical wear of the garment.

Yet a further object of the invention is to bring about the reduction in this risk without impairing the convenience for the wearer of the garment. In order to achieve a secure barrier, the adhesive must adhered tightly to the skin, and the strength of the adhesive must be sufficiently high for the adhesive to withstand the movements that occur as a result of movements in the face or by the body.

When they are removed, traditional adhesives are accompanied by large areas of the stratum corneum, that is to say the top layer of the skin, which gives rise to redness and, in certain cases, irritation of the skin. When the surgical garment is a garment that is replaced between every operation and is worn daily by hospital personnel, this imposes extra high demands on the adhesive.

Comfort is an important characteristic of surgical gowns, face masks and other garments intended for operating personnel. As it may be difficult to find the right positioning for the garment at the first attempt, it is an important characteristic for the garment to be capable of being reapplied without losing its adhesive strength. If the level of adhesion of the adhesive is significantly lower in the event of reapplication, there is a considerable risk of the barrier function being impaired.

Problems are very often encountered with surgical gloves slipping down during the operation. Because today's gloves are pulled over the wristband and the lower part of the gown, the glove can slide down easily, for example when the sleeve is extended. One way of counteracting this is to put on the surgical gloves first instead, and the gown on top, and an adhesive that is present inside the sleeve of the gown can then be applied to the glove in order to hold the gown in place and seal between the glove and the gown.

The object of the present invention is to solve these problems and to make available a surgical garment which adheres securely around openings, provides a secure barrier, is removable without the risk of damage being caused to the skin, is capable of reattachment to the skin without the risk of the adhesive strength of the adhesive having reduced excessively, and offers increased comfort to the wearer.

US 2003/0136410 A1 and US 2004/0055078 A1 disclose face masks sealed to skin by adhesive, US 5,797,146 discloses a breath deflector attached to the skin by an adhesive and WO 03/079920 A1 discloses a surgical drape attached to the skin by a silicone elastomer or a hot melt adhesive.

### DISCLOSURE OF INVENTION

These objects are achieved with a garment, such as gowns, face masks, gloves and headwear, for personnel involved in surgical operations, characterized by a skin friendly adhesive for the sealing attachment of parts of the garment to the skin of a person wearing the garment. By means of such a skin friendly adhesive, a barrier is formed in openings in such a garment, such as the neckline in a surgical gown or the periphery of a face mask, which prevents air, blood and the like from penetrating through the opening. The fact that the adhesive is skin friendly also does not reduce the convenience for the wearer.

In a preferred embodiment, the part of the garment provided with adhesive is leakproof in accordance with the MHC Leakage Test with a groove depth of 50 micrometres, 75 micrometres, 150 micrometres and 200 micrometres.

The adhesive also has a weight per unit area of 50 g/m² or more and a softness greater than 10 mm. In a preferred variant, the adhesive has a weight per unit area of 80 g/m² or more and a softness greater than 10 mm. The adhesive can have a weight per unit area of 200 g/m² or more and a softness greater than 10 mm. The adhesive can with advantage have a softness greater than 12 mm, and preferably greater than 14 mm.

The adhesive consists of a hot-melt adhesive or a silicon elastomer.

In addition, the strength of the attachment of the adhesive to the skin is greater than 0.5 N/25 mm, preferably greater than 1.0 N/25 mm, and more preferably greater than 1.5 N/25 mm, and in the case of reapplication to the skin, the adhesive strength of the adhesive is reduced by less than 40%, preferably by less than 30%, and best of all by less than 15%.

When measured by means of the SCT (Spectroscopic Colour Test), damage to the Stratum Corneum following the removal of a garment attached to the skin is less than 40%, preferably less than 30%, preferably less than 20%, and more preferably less than 10%, on that part of the skin that was covered by the adhesive.

The invention also relates to the use of a skin friendly adhesive for the removable and re-attachable attachment of a face mask to a wearer. In the case of such use, the risk of air-borne bacteria being able to exit from or enter the area inside the face mask is radically reduced. Furthermore, the elastic bands that are used to attach the face mask around the ears of a wearer are superfluous in conjunction with the use of such an adhesive, which increases the convenience for the wearer. The formable elements of the face mask, which are used to fit the face mask to the shape of the nose of a wearer, can also be replaced by a skin friendly adhesive, which further increases the convenience for the wearer.

The invention also relates to the use of a skin friendly adhesive for the attachment of an item of surgical headwear to a wearer in a manner that is sealed to the skin and re-attachable, the use of a skin friendly adhesive for the attachment of the neckline and/or the ends of the sleeves of a surgical gown to the skin of a wearer in a manner that is tightly sealing and re-closable, and the use of a skin friendly adhesive for the attachment of a part of a surgical glove, which extends around the forearm of a wearer, to the skin of the wearer in a manner that is tightly sealing and re-closable.

### BRIEF DESCRIPTION OF DRAWINGS

The invention is now described below with reference to the accompanying Figures, in which:
Fig. 1 illustrates schematically a surgical gown in accordance with a first preferred embodiment of the invention;
Fig. 2 illustrates schematically a face mask in accordance with a second preferred embodiment of the invention;
Fig. 3 illustrates schematically the measurement of the strength of the adhesive attachment to the skin;
Fig. 4 illustrates a cone used for the measurement of the softness;
Fig. 5 illustrates a method of measurement for measuring the softness;
Figs. 6-12 illustrate the MHC Leakage Test;
Fig. 13 shows a graph indicating the relationship between the weight per unit area and the softness in order to obtain sealing in accordance with the MHC Leakage Test, and
Figs. 14-16 show the MHC Leakage Test performed on known products and on a product in accordance with the invention for different groove depths.

### MODE(S) FOR CARRYING OUT THE INVENTION

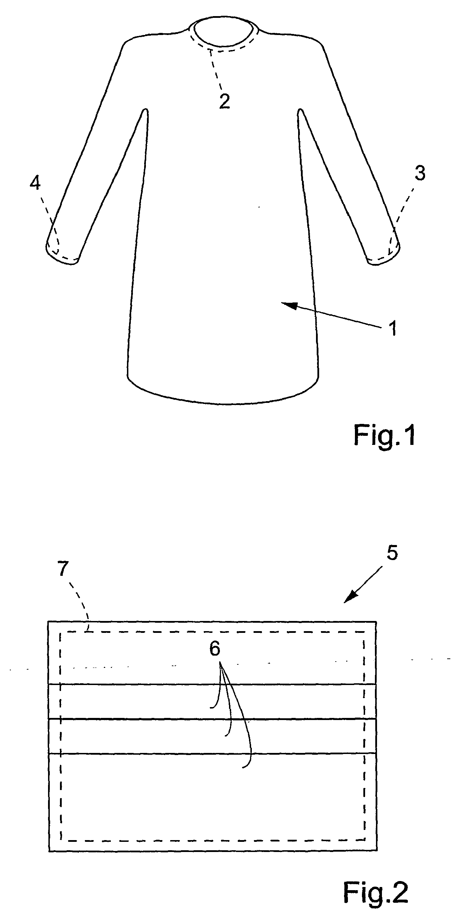

The surgical gown 1 illustrated schematically in Figure 1 is constructed in a conventional manner and differs from conventional surgical gowns only in the respect that it is coated on the inside with a skin friendly adhesive in the areas 2, 3 and 4. The area 2 in this case extends around at least the front part of the neckline of the gown, and the areas 3 and 4 extend around the ends of the sleeves of the gown. When wearing a surgical gown of this kind, the adhesive coatings seal the openings leading in to the wearer's body constituted by the sleeves and the neckline.

Illustrated schematically in Figure 2 is a face mask 5 in a plan view with its outside facing towards the observer. The face mask comprises in a conventional fashion a number of folds 6, which are sealed at the outer edges of the face mask. The fold 6 can be folded out in a manner resembling a bellows, so that the face mask assumes a three-dimensional, triangular form.

In accordance with the invention, the face mask also comprises a peripheral coating 7 of a skin friendly adhesive. The presence of a skin friendly adhesive for the purpose of attaching the face mask makes it possible to dispense with the elastic bands that are fitted to face masks for attachment around the ears in order to support the face mask by that means. Metal wires or the like are no longer required, moreover, in order to form the upper part of the mask to the shape of the root of the nose, and this is now effected with the help of that part of the peripheral adhesive coating that extends transversely over and past the root of the nose of a wearer. A face mask in accordance with the invention makes closer contact with the wearer's skin than is the case with currently available face masks, and it functions more effectively as a result. A further, not insignificant advantage is that a face mask in accordance with the invention is more comfortable to wear than currently available face masks. In addition, the whole of the upper edge of the face mask will be caused to seal against the wearer's skin by the agency of the adhesive, which prevents exhaled air from being able to flow upwards and cause condensation to form on the spectacles of a wearer. A further major advantage is that the adhesive attached to the skin counteracts friction and reduces the risk of skin particles becoming detached and contaminating the patient's operation wound. A healthy person can shed thousands of skin particles, which carry bacteria on them, every minute when moving around.

The adhesive in the coating must, as previously mentioned, be skin friendly and in addition must permit the removal of the garment without causing damage to the skin around the attachment area. These requirements mean that those types of pressure-sensitive adhesives that are customarily used as adhesive coatings for surgical drapes and wound care products cannot be used. Such adhesives often attach themselves to the skin so strongly that parts of the Stratum Corneum, that is to say the uppermost layer of the skin, become stuck to the adhesive and are pulled away from the skin when the attachment of the product is released. Certain of these adhesives are attached so strongly that even hairs are pulled out when the attachment is released. The adhesive coating intended for sealing can exhibit different appearances and can consist of solid or broken lines, points or patterns of various kinds. It can also be executed as an unbroken strip.

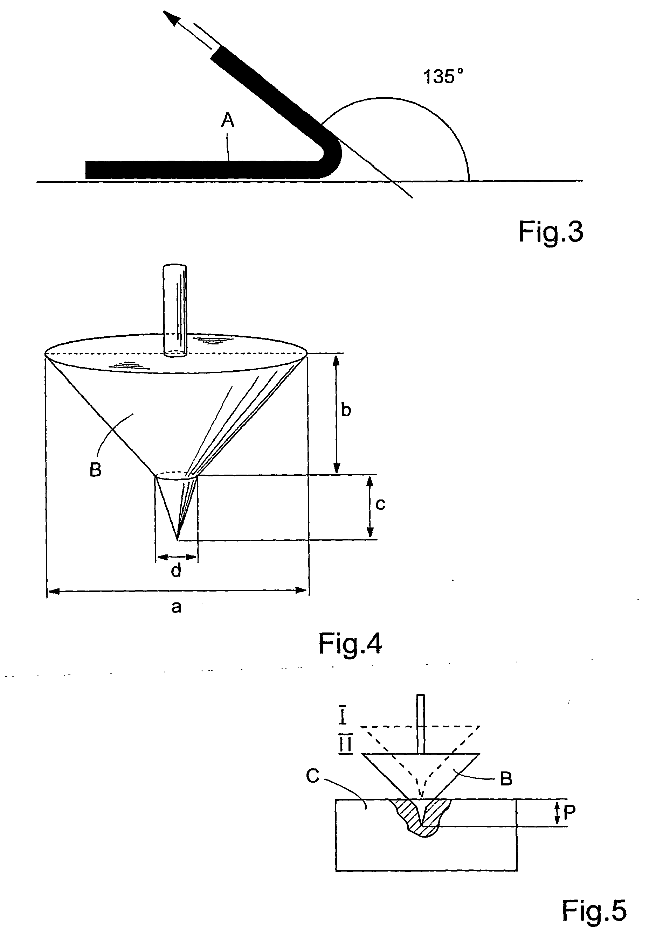

Because the characteristics of the skin vary from person to person, the adhesion capacity of the adhesive coating to the skin naturally also varies for different persons. The values of the forces of adhesion to the skin of an adhesive, as indicated below, must be measured by means of a method of the kind illustrated schematically in Figure 3. Strips A of the adhesive-coated material to be tested with a width of 25 mm are applied to the skin on the back of at least ten healthy persons of varying ages and sexes and are left in place on the skin for 6 hours. The strips A are then pulled off at a rate of 25 mm/sec, and the removal force F1 is measured. The angle of removal, that is to say the obtuse angle that is formed between the surface of the skin and the removed part of the strip A, must be 135°. The force of adhesion to the skin of the measured adhesive comprises the mean value of the force F1. Adhesives suitable for use in products in accordance with the invention must exhibit an adhesive strength of at least 0.5 N/25 mm.

In order to ensure that the adhesive does not damage the skin, the damage to the Stratum Corneum after the removal of a draping product attached to the skin shall, when measured by means of the SCT (Spectroscopic Colour Test), be less than 30%, preferably less than 20%, and more preferably less than 10%, on that part of the skin that was covered by the adhesive.

The SCT measurement shall be performed by the method described in detail in P.J. Dykes, R. Heggie, S.A. Hill, "Effects of adhesive dressings on the stratum corneum of the skin", Journal of Wound Care, February, Vol. 10, No. 2, 2001, to which reference is made for more detailed information. The SCT measurement must be performed on at least ten persons of different sexes and with healthy skin, and must proceed by the following method. The skin at the centre of the test area is first stained by the application of a 12 mm Finn chamber of aluminium containing an 11 mm filter paper disc moistened with 0.03 ml of a 1% aqueous solution of methylene blue. The Finn chamber is held against the skin for 60 minutes. This is sufficient to bring about the uniform staining of the outermost layers of the stratum corneum. The test strips are then applied to the stained areas of the skin on the test individuals and are left in place for 72 hours. Once the test strips have been removed after 72 hours, the stratum corneum is removed by means of the "skin surface biopsy procedure", as described in R. Marks, R.P.R. Dawber, "Skin surface biopsy: an improved technique for the examination of the horny layer", Br J Dermatol 1971:84:117-123, and to which reference is made for more detailed information. The biopsies are then cut into quite small pieces and placed in glass test tubes containing 2 ml of dimethylsulfoxide (DMSO). The glass test tubes are then shaken every 10-15 minutes during a period of 2 hours in order to ensure that the colour extraction is complete. The dimethylsulfoxide extract is then centrifuged at 1000 g for 10 minutes in order to remove all fragments of the stratum corneum. One millilitre of the dimethylsulfoxide is then transferred to a plastic vessel for measurement of the optical density. The optical density is measured with a spectrophotometer. A reference vessel containing dimethylsulfoxide is first scanned within the range 550-800 nm. An extracted skin biopsy from a stained area of the skin is then scanned to determine the maximum absorption. All subsequent measurements are performed at the wavelength for maximum absorption. The results are indicated as units of optical density and are stated as a percentage of damaged Stratum Corneum compared with a reference sample of adjacent undamaged Stratum Corneum.

Table 1 below illustrates the damage caused to the Stratum Corneum by the removal of adhesive from the skin, measured by the abovementioned spectroscopic staining method (SCT) for a number of different, previously disclosed products provided with adhesive: Allevyn from Smith & Nephew, Hull, Great Britain; Tielle hydropolymer dressing from Johnson & Johnson, Gargrave, Great Britain; Duoderm Extra Thin from ConvaTec Ltd., Deeside, Great Britain; Mepilex Border from Mölnlycke Health Care AB, Gothenburg, Sweden, and Biatain from Coloplast, Humlebæk, Denmark.

**Table 1**

| | Damage to Stratum Corneum |
|---|---|
| | (%) |
| Allevyn adhesive | 96.4 |
| Tielle hydropolymer adhesive | 90.9 |
| Duoderm | 81.8 |
| Mepilex Border adhesive | -1.8 |
| Biatain adhesive | 87.3 |

It can be appreciated from Table 1 that only the adhesive supplied by Mepilex Border, which consists of a silicon adhesive, Silgel 612 elastomer from Wacker Chemie GmbH, Germany, meets the requirements stipulated above. The negative value of the skin damage measured by the SCT test method on skin with Mepilex Border adhesive is probably an effect of the distribution of the measurement data, although it may also relate to the fact that the adhesive acts as a means of protection against the natural abrasion of skin cells compared with the reference sample that is only covered by gauze during the measurement period.

It has been found that strips of an adhesive, which possesses an adhesive strength of 0.5 N/25 mm and, when measured by means of the SCT (Spectroscopic Colour Test), reveals a level of damage to the Stratum Corneum, after removal of a draping product attached to the skin, of less than 10% on that part of the skin that was covered by the adhesive, can also be applied to sensitive skin and removed therefrom without causing damage or irritation to the skin.

Measurement of the strength of adhesion to the skin with re-application is performed by the following method. Test strips are applied to the back of ten persons of varying ages and sexes with healthy skin. After two minutes, the strips are pulled off in the same way as described above with reference to Figure 3 at a rate of 25 mm/sec, and the removal force Ffirst is measured. The angle of removal must be 135°. The strip is then applied to the skin once more in an untouched area on the back, and measurement of the removal force is repeated after two minutes, in conjunction with which the removal force Fsecond is obtained. The reduction in the removal force in conjunction with the second and the first removals must be less than 40%, preferably less than 30%, and more preferably less than 20%, and the removal force Fsecond in conjunction with the second removal must exceed 0.5 N/25 mm, preferably 1.0 N/25 mm, and more preferably 1.2 N/25 mm.

A measurement of this kind was performed for adhesive-coated strips taken from the following commercially available products: Klinidrape® Universal Set Basic, Item No. 698740, from Mölnlycke Health Care AB, Gothenburg, Sweden; Allegiance Convertors, REF 2915CE from McGaw Park, Illinois, USA, and 3M Steri-Drape, 9000 from 3M, St. Paul, Minnesota, USA, and for a strip of Klinidrape® material, to which a strip of polyurethane had been laminated and coated with the Silgel 612 elastomer from Wacker Chemie AG, Germany. The result of the measurements is presented in Table 2 below.

**Table 2**

| | Silicon adhesive | Klinidrape adhesive, U-set | Allegiance adhesive, U-set | 3M adhesive, U-set |
|---|---|---|---|---|
| Fₘₐₓ first (N/25 mm) | 1.71 | 0.72 | 0.80 | 0.82 |
| Fₘₐₓ second (N/25 mm) | 1.44 | 0.35 | 0.37 | 0.41 |
| Reduction in adhesive strength (%) | 16 | 51 | 54 | 50 |
| Fₘₑₐₙ first (N/25 mm) | 1.14 | 0.50 | 0.52 | 0.60 |
| Fₘₑₐₙ second (N/25 mm) | 1.00 | 0.24 | 0.24 | 0.28 |
| Reduction in adhesive strength (%) | 12 | 51 | 53 | 53 |

It can be appreciated from this measurement that Silgel 612 is able to function excellently as an adhesive for a product in accordance with the invention.

It has been found that a silicon elastomer with an adhesive strength of 1.5 N/25 mm also satisfies the requirement, when measured by means of the SCT (Spectroscopic Colour Test), that the level of damage to the Stratum Corneum, after removal of a draping product attached to the skin, is less than 10% on that part of the skin that was covered by the adhesive. Such an elastomer is thus very suitable for use in products in accordance with the invention.

In order to reduce the necessary unit of width/length of the adhesive coating, and to increase the margin of safety during use, the strength of the adhesive attachment to the skin of the adhesive coating is with advantage greater than 1.0 N/25 mm, and preferably greater than 1.2 N/25 mm.

Adhesives that are suitable for use in accordance with the present invention must exhibit a softness that exceeds 10 mm measured by means of a method based on ASTM D 937 and ASTM D 51580. Certain deviations, as can be appreciated below, have been made. Figures 4 and 5 illustrate this modified method of measuring the softness of an adhesive by causing a cone B with a weight of 62.5 g to penetrate downwards by the effect of gravity into a 30 mm thick test piece C of the adhesive of which the softness is to be determined. The test piece is produced by filling a cylindrical glass container having an internal diameter of 60 mm, and an internal height of 35-40 mm, with adhesive to a depth of 30 mm. The cone used is illustrated in Figure 4 and has the following dimensions: a=65 mm, b=30 mm, c=15 mm and d=8.5 mm. In the performance of the method for measurement of the softness, the cone B is first lowered down into a position I, which is illustrated with broken lines in Figure 5, and in which the tip of the cone just touches the surface of the test piece C. The cone B is then released, so that it is able to penetrate downwards into the test piece C by the effect of gravity. The number of millimetres by which the tip B of the cone C has penetrated into the test piece C after 5 seconds is measured and constitutes the penetration value P, the value of which is greater in proportion to the softness of the test piece. The penetration value P represents the softness index used in the present invention. A PNR 10 penetrometer supplied by Sommer & Runge KG, Germany, is used in the performance of the method.

It has also been found that, in the case of soft, skin friendly adhesives, which form barriers preventing fluid from flowing through them, fluid is capable of leaking through these barriers via cracks in the skin, folds in the skin or other unevenness in the skin. This leakage can give rise to the propagation of bacteria, which in turn can lead to wound infections.

Surprisingly, it has also been found that the abovementioned risk of leakage can be eliminated, or at least significantly reduced, for a soft, skin friendly adhesive if the weight per unit area of the adhesive and/or its softness is increased.

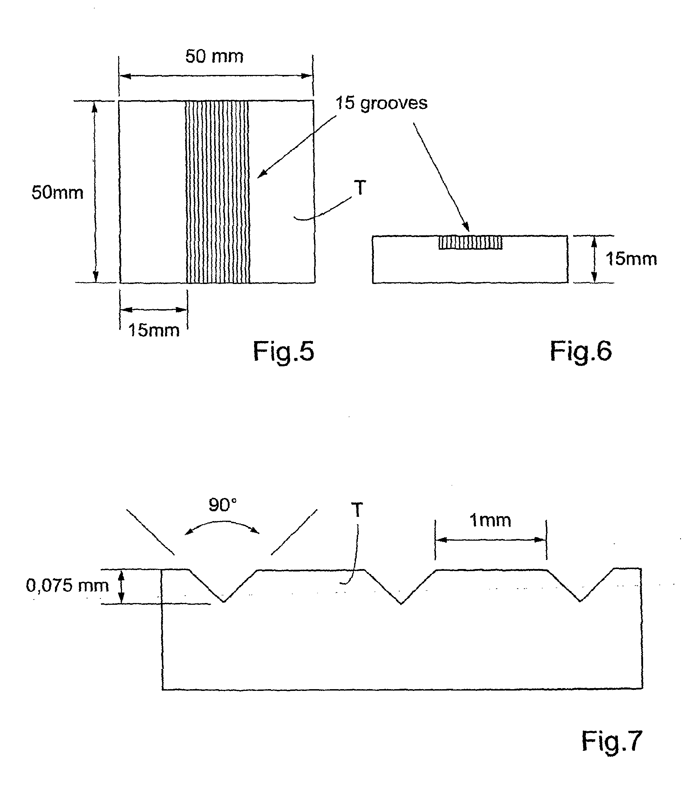

The method described below with reference to Figures 6-12, known as the MHC Leakage Test, was developed by the applicants for the purpose of determining whether or not a coating of a soft, skin friendly adhesive is leakproof. Specimens with a size of 30 x 30 mm from the product to be tested are taken, and a circular hole (d = 12 mm) is removed from the centre of the samples by punching. A coloured test fluid is prepared by mixing 0.2% by weight of Patentblått V (from VWR International, Sweden) and 0.1% by weight of Teepol Gold (from Teepol Products, UK) with de-ionized water. An aluminium test plate having dimensions of 15 x 50 x 50 mm and provided with 15 milled grooves is made; see Fig. 6 (viewed from above) and Fig. 7 (viewed from the side). For a more detailed description of the form of the grooves, see Fig. 8 (section through the plate, viewed from the side). Illustrated in Figure 8 are grooves with a depth of 75 micrometres, although other groove depths can be used in the test depending on what depth of cracks or folds in the skin the product is intended to seal.

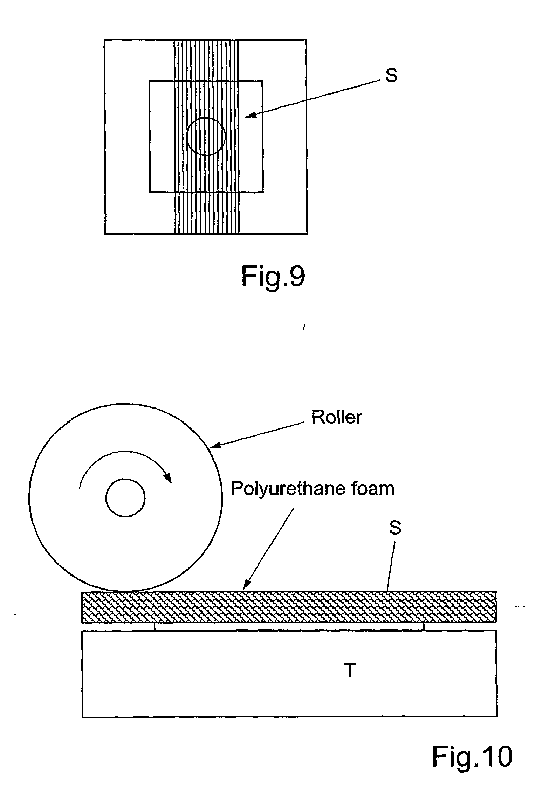

A specimen is then carefully positioned centrally above the grooves of the test plate in such a way that no air bubbles are produced between the test plate and the specimen; see Fig. 9. No pressure may be exerted on the sample when it is positioned against the plate, so that, in the event that air bubbles are produced, these must not be forced away with the help of the fingers, but the sample must be raised and repositioned, or scrapped.

A piece of polyurethane foam (L00562-6, 1.6 mm from Rynel, Inc., Boothbay, ME, USA) having dimensions of 50 x 50 mm is then placed above the sample and the test plate. A mangle made of metal (44 mm wide, r = 48 mm, weight = 995 g) is then rolled over the foam and the specimen at a speed of 5 mm/second; see Fig. 10. The mangle is rolled back and forth once over the sample.

The piece of foam is removed from the sample, and 65 µl of the test fluid are placed in the hole on the specimen with the help of a pipette. The test fluid is distributed uniformly in the hole with the help of the tip of the pipette, so that the fluid reaches every point on the edge of the sample. A stop watch is started as soon as all the test fluid is uniformly distributed in the hole. After 30 minutes, a picture is taken with a digital camera of the specimen and the test fluid placed on the test plate together with a calibrated ruler.

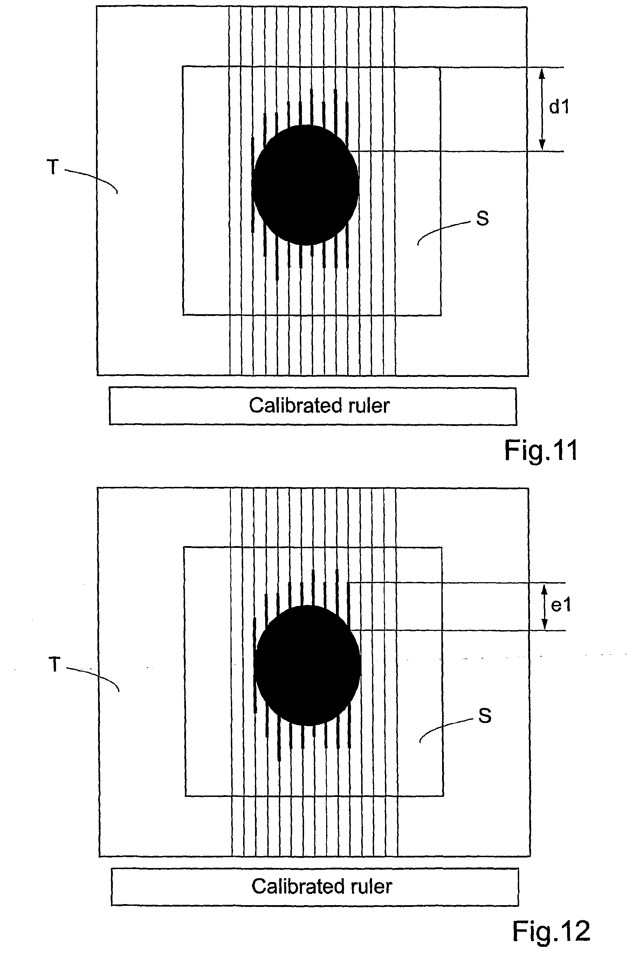

The photograph is used to measure the following distances. For all the grooves that are in contact with the hole on the sample, that is to say in all the grooves into which fluid may be expected to penetrate, the distance d from the edge next to the hole to the edge on the end of the sample is measured, see Fig. 11, which indicates this distance dl for one of the grooves. All these distances d are then added together, and they constitute the total distance for which it is possible for the sample to leak. After this, the distance e for which the test fluid has leaked in all the grooves on the plate is measured; see Fig. 12, which shows the distance e1 for one of the grooves. The combined length of all the distances e represents the total leakage distance.

Finally, the leakage is obtained by dividing the combined leakage distance by the total distance for which it is possible for the sample to leak. This quotient is then converted into a percentage by multiplying it by 100. The evaluation of the sealing is performed as follows: Result > 10% leakage, regarded as leakage. Result ≤ 10% leakage, regarded as sealing.

Note that, between each measurement on the test plate, the plate must be cleaned in the following way. The plate is first rinsed with water, and it is then washed with n-heptane. It is important to ensure that no adhesive residues remain in the grooves on the plate, and a soft material of the nonwoven compress type (Mesoft, Mölnlycke Health Care) can be dipped in n-heptane and used to rub away adhesive residues in the grooves on the plate. Finally, the plate must be left to dry in the air before it can be reused.

Other solvents may be used for adhesives that are not soluble in n-heptane.

If the security against leakage is to be tested for products that are not transparent, a transparent plastic film is coated with the adhesive which the product contains, after which specimens with an area of 30 x 30 mm are punched from this material. The abovementioned plastic film must be selected so that its bending length corresponds to the bending length of the carrier in the non-transparent product that is to be tested measured in accordance with the "Determination of bending length" method, ISO 9073-7:1995. The MHC Leakage Test is then performed, as described above.

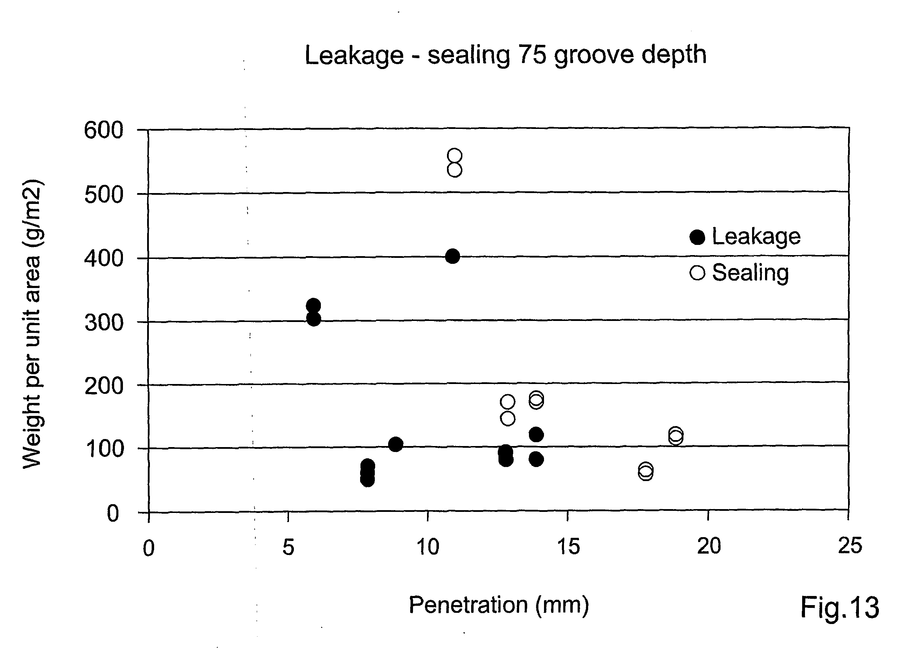

The MHC Leakage Test with a groove depth of 75 micrometres was performed on a polyurethane film with a thickness of 25 +/- 5 micrometres, which was coated with a Silgel 612 silicon elastomer supplied by Wacker Chemie GmbH, Germany, with different softness values and weights per unit area. The results are shown in Figure 13.

The results clearly indicate that there is a link between the softness (penetration) and the weight per unit area of the silicon elastomer. The softer the silicon elastomer, the smaller the weight per unit area required for sealing. The result points to the fact that, for a sufficient number of measurements, it is possible to produce a curve that indicates exactly the minimum weight per unit area that is required at a given softness to ensure sealing against the skin. The results make it clear that such a curve has a steep incline initially, that is to say in the case of less soft adhesives, after which it levels out. It is obvious that, at softness values below 10 mm, it is difficult, and perhaps even impossible, to achieve fluid-tight products with the selected adhesive, whereas, at softness values in the order of 20 mm, a weight per unit area of 50 g/m² may be sufficient to achieve sealing.

When using other adhesives, it can be expected that the values will change, but that the qualitative appearance of the curve will remain the same.

The carrier is an important part of the product, and this, too, has a major effect on the degree of sealing, especially in the case of low weights per unit area for the adhesive coating. The more sensitive the material, the better the carrier is capable of following the folds in the skin and, as a consequence of this, a smaller weight per unit area is required by a soft adhesive. If the carrier is rigid, the flexibility and the sensitivity must be present to a higher degree in the adhesive bulk, which calls for a greater adhesive bulk with a higher weight per unit area. A rigid carrier thus requires a higher weight per unit area for the adhesive coating than a less rigid carrier in order to produce sealing.

It has also emerged that an increased weight per unit area increases the adhesion in a product. External shearing forces can then be absorbed and distributed in the adhesive layer, instead of influencing the attachment between the patient's skin and the adhesive. This reduces the risk that the product may work loose fully or partially under external loading, thereby contributing to a reduced risk of post-operative infection for the patient.

Shearing forces which act on the self-adhesive attachment can also give rise to a separation between the different layers of skin. This results in the formation of a blister, that is to say a well-defined accumulation of serous fluid. The blisters occur along the self-adhesive attachment and are usually located at different depths in the epidermis or at the boundary between the epidermis and the dermis. The shearing forces which act on the attachment can arise, on the one hand, from external loading, but also as a result of a swelling occurring during the operation. By increasing the weight per unit area on the adhesive, and by utilizing the softest possible adhesive, that is to say a high value for the penetration, the adhesive layer will absorb a large proportion of the shearing forces that would otherwise have acted upon the skin.

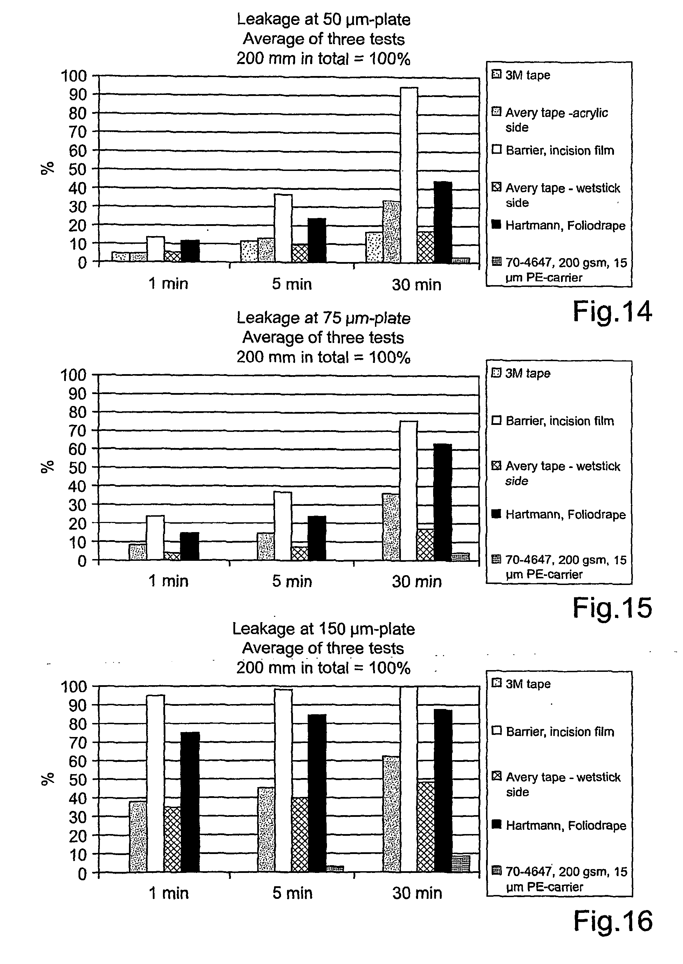

Figures 14-16 illustrate different products tested by means of the MHC Leakage Test with groove depths of 50, 75 and 150 micrometres respectively and illustrate the leakage after 1 minute, 5 minutes and 30 minutes. The tested products were 3M™ Hi-Tack Double Coated Medical Tape, Product Number 1517 supplied by 3M, USA; MED 6370U, Avery Dennison™, Acrylic Adhesive supplied by Avery Dennison, USA; Barrier Flex supplied by Neschen AG, Germany; MED 6370U Avery Dennison™, Wetstick Adhesive supplied by Avery Dennison, USA; Foliodrape®, Hartmann, Transparent OP-tape, No. 258 542, LOT 348 01705, Exp. Date 2008-12 supplied by Hartmann, Germany, and DISPOMELT 70-4647, 200 gsm ±20 gsm supplied by National Starch & Chemical, USA, PE-carrier (15 µm).

As can be appreciated from Figures 14-16, all the samples leaked, apart from the polyethylene film coated with the DISPOMELT 70-4647 hot-melt adhesive at all groove depths. The soft-melt adhesive had a softness of 14.7 mm.

Of the tests carried out, it can thus be appreciated that it is possible to produce leakproof products in conjunction with the use of soft adhesives by increasing the weight per unit area of the adhesive coating. The test also shows that the polyethylene film coated with the DISPOMELT 70-4647 hot-melt adhesive is leakproof for the majority of cracks in the skin or folds in the skin that are encountered in normal skin.

The products proposed in the present invention are often supplied packed in sterile conditions, which means that the adhesives used must be capable of being sterilized, as must other components of such articles, of course.

A skin friendly adhesive coating can naturally also be used to attach types of garments other than gowns and face masks for personnel involved in surgical operations to the skin of the wearer. Examples of other applications are headwear and gloves. For gloves, the coating is applied to the inside in that part of the glove that extends above the wrist around the forearm of the wearer. For gloves, the primary function of the adhesive coating is to prevent that part of the glove that extends above the hand from slipping down from the forearm. Face masks are also used by persons other than the personnel involved in surgical operations, for example by dentists, where sterility is not a requirement. Under such conditions, tightness against leakage is not as important, and skin friendliness and resealability are the critical factors for the functionality of the face mask. Skin friendly adhesive coating can also be used for the attachment of a visor or protective goggles.

The embodiments described here can naturally be modified within the scope of the invention. For example, the form and the execution of the surgical gown can be varied, as can the form of the face mask. Moreover, adhesives other than those referred to above can be used, provided that they satisfy the stipulated requirements in respect of their adhesive strength and skin friendliness. The scope of the invention must accordingly only be restricted by the wording of the accompanying Patent Claims.

## Claims

1. A garment, such as gowns (1), face masks (5), gloves and headwear for personnel involved in surgical operations, comprising a skin friendly adhesive (7) for the sealing attachment of parts of the garment to the skin of a person wearing the garment, **characterized in that** the adhesive consists of a silicone elastomer or hot-melt adhesive and has a weight per unit area of 50 g/m² or more and a softness between 20-10 mm (measured by means of a method based on ASTM D 937 and ASTM D 51580), the softer the adhesive, the smaller weight per unit area required for sealing.

2. A garment in accordance with Claim 1, **characterized in that** the adhesive (7) is leakproof in accordance with the MHC Leakage Test with a groove depth of 50 micrometres.

3. A garment in accordance with Claim 2, **characterized in that** the adhesive (7) is leakproof in accordance with the MHC Leakage Test with a groove depth of 75 micrometres.

4. A garment in accordance with Claim 3, **characterized in that** the adhesive (7) is leakproof in accordance with the MHC Leakage Test with a groove depth of 150 micrometres.

5. A garment in accordance with Claim 4, **characterized in that** the adhesive (7) is leakproof in accordance with the MHC Leakage Test with a groove depth of 200 micrometres.

6. A garment in accordance with one or other of Claims 1-5, **characterized in that** the adhesive (7) has a weight per unit area of 80 g/m² or more and a softness greater than 10 mm (measured by means of a method based on ASTM D 937 and ASTM D 51580).

7. A garment in accordance with one or other of Claims 1-5, **characterized in that** the adhesive (7) has a weight per unit area of 200 g/m² or more and a softness greater than 10 mm (measured by means of a method based on ASTM D 937 and ASTM D 51580).

8. A garment in accordance with one or other of Claims 1-6, **characterized in that** the adhesive (7) has a softness greater than 12 mm (measured by means of a method based on ASTM D 937 and ASTM D 51580).

9. A garment in accordance with Claim 8, **characterized in that** the adhesive (7) has a softness greater than 14 mm (measured by means of a method based on ASTM D 937 and ASTM D 51580).

10. The use of a skin friendly adhesive (7) consisting of a silicone elastomer or hot-melt adhesive and having a weight per unit area of 50 g/m² or more and a softness between 20 - 10 mm (measured by means of a method based on ASTM D 937 and ASTM D 51580), the softer the adhesive, the smaller weight per unit area required for sealing, for the removable and re-attachable attachment of a face mask (5) to a wearer.

11. The use of a skin friendly adhesive consisting of a silicone elastomer or hot-melt adhesive and having a weight per unit area of 50 g/m² or more and a softness between 20 - 10 mm (measured by means of a method based on ASTM D 937 and ASTM D 51580), the softer the adhesive, the smaller weight per unit area required for sealing, for the attachment of an item of surgical headwear to a wearer in a manner that is sealed to the skin and re-attachable.

12. The use of a skin friendly adhesive consisting of a silicone elastomer or hot-melt adhesive and having a weight per unit area of 50 g/m² or more and a softness between 20 - 10 mm (measured by means of a method based on ASTM D 937 and ASTM D 51580), the softer the adhesive, the smaller weight per unit area required for sealing, for the attachment of the neckline (2) and/or the ends of the sleeves (3,4) of a surgical gown to the skin of a wearer in a manner that is sealing and re-closable.

13. The use of a skin friendly adhesive consisting of a silicone elastomer or hot-melt adhesive and having a weight per unit area of 50 g/m² or more and a softness between 20 - 10 mm (measured by means of a method based on ASTM D 937 and ASTM D 51580), the softer the adhesive, the smaller weight per unit area required for sealing, for the attachment of a part of a surgical glove, which extends around the forearm of a wearer, to the skin of the wearer in a manner that is sealing and re-closable.

## Patentansprüche

1. Kleidungsstück, wie beispielsweise Kittel (1), Gesichtsmasken (5), Handschuhe und Kopfbedeckung für Personal, das an chirurgischen Operationen beteiligt ist, das ein hautfreundliches Haftmittel (7) zum abdichtenden Befestigen von Teilen des Kleidungsstücks an der Haut der Person, die das Kleidungsstück trägt, umfasst, **dadurch gekennzeichnet, dass** das Haftmittel aus einem Silikonelastomer oder einem heißschmelzenden Haftmittel besteht und ein Gewicht pro Einheitsfläche von 50 g/m² oder mehr und eine Weichheit zwischen 20 und 10 mm (gemessen durch ein Verfahren auf Basis von ASTM D937 und ASTM D51580) hat, je weicher das Haftmittel, desto weniger Gewicht pro Einheitsfläche wird für das Abdichten benötigt.

2. Kleidungsstück gemäß Anspruch 1, **dadurch gekennzeichnet, dass** das Haftmittel (7) leckdicht ist gemäß dem MHC-Leakage-Tests mit einer Kerbentiefe von 50 µm.

3. Kleidungsstück gemäß Anspruch 2, **dadurch gekennzeichnet, dass** das Haftmittel (7) leckdicht ist gemäß dem MHC-Leakage-Tests mit einer Kerbentiefe von 75 µm.

4. Kleidungsstück gemäß Anspruch 3, **dadurch gekennzeichnet, dass** das Haftmittel (7) leckdicht ist gemäß dem MHC-Leakage-Tests mit einer Kerbentiefe von 150 µm.

5. Kleidungsstück gemäß Anspruch 4, **dadurch gekennzeichnet, dass** das Haftmittel (7) leckdicht ist gemäß dem MHC-Leakage-Tests mit einer Kerbentiefe von 200 µm.

6. Kleidungsstück gemäß einem oder mehreren der Ansprüche 1 bis 5, **dadurch gekennzeichnet, dass** das Haftmittel (7) ein Gewicht pro Einheitsfläche von 80 g/m² oder mehr und eine Weichheit größer als 10 mm (gemessen durch ein Verfahren auf Basis von ASTM D937 und ASTM D51580) hat.

7. Kleidungsstück gemäß einem oder mehreren der Ansprüche 1 bis 5, **dadurch gekennzeichnet, dass** das Haftmittel (7) ein Gewicht pro Einheitsfläche von 200 g/m² oder mehr und eine Weichheit größer als 10 mm (gemessen durch ein Verfahren auf Basis von ASTM D937 und ASTM D51580) hat.

8. Kleidungsstück gemäß einem oder mehreren der Ansprüche 1 bis 6, **dadurch gekennzeichnet, dass** das Haftmittel (7) eine Weichheit größer als 12 mm (gemessen durch ein Verfahren auf Basis von ASTM D937 und ASTM D51580) hat.

9. Kleidungsstück gemäß Anspruch 8, **dadurch gekennzeichnet, dass** das Haftmittel (7) eine Weichheit größer als 14 mm (gemessen durch ein Verfahren auf Basis von ASTM D937 und ASTM D51580) hat.

10. Verwendung eines hautfreundlichen Haftmittels (7), bestehend aus einem Silikonelastomer oder einem heißschmelzenden Haftmittel, mit einem Gewicht pro Einheitsfläche von 50 g/m³ oder mehr und einer Weichheit zwischen 20 und 10 mm (gemessen durch ein Verfahren auf Basis von ASTM D937 und ASTM D51580), je weicher das Haftmittel, desto weniger Gewicht pro Einheitsfläche wird für das Abdichten benötigt, für das entfernbare und wieder anbringbare Anlegen einer Gesichtsmaske (5) für den Träger.

11. Verwendung eines hautfreundlichen Haftmittels, bestehend aus einem Silikonelastomer oder einem heißschmelzenden Haftmittel, mit einem Gewicht pro Einheitsfläche von 50 g/m³ oder mehr und einer Weichheit zwischen 20 und 10 mm (gemessen durch ein Verfahren auf Basis von ASTM D937 und ASTM D51580), je weicher das Haftmittel, desto weniger Gewicht pro Einheitsfläche wird für das Abdichten benötigt, für das Festmachen eines chirurgischen Kopfbedeckungsstückes am Träger auf eine zur Haut dichtschließende und wiederanbringbare Weise.

12. Verwendung eines hautfreundlichen Haftmittels, bestehend aus einem Silikonelastomer oder einem heißschmelzenden Haftmittel, mit einem Gewicht pro Einheitsfläche von 50 g/m³ oder mehr und einer Weichheit zwischen 20 und 10 mm (gemessen durch ein Verfahren auf Basis von ASTM D937 und ASTM D51580), je weicher das Haftmittel, desto weniger Gewicht pro Einheitsfläche wird für das Abdichten benötigt, für das Anbringen des Halsausschnitts (2) und/oder der Enden der Ärmel (3, 4) eines chirurgischen Kittels an der Haut des Trägers auf dichtschließende und wiederverschließbare Weise.

13. Verwendung eines hautfreundlichen Haftmittels, bestehend aus einem Silikonelastomer oder einem heißschmelzenden Haftmittel, mit einem Gewicht pro Einheitsfläche von 50 g/m³ oder mehr und einer Weichheit zwischen 20 und 10 mm (gemessen durch ein Verfahren auf Basis von ASTM D937 und ASTM D51580), je weicher das Haftmittel, desto weniger Gewicht pro Einheitsfläche wird für das Abdichten benötigt, für das Anbringen eines Teils eines chirurgischen Handschuhs, der sich um den Unterarm des Trägers erstreckt, an der Haut des Trägers auf dichtschließende und wiederverschließbare Weise.

## Revendications

1. Vêtement, tel que des blouses (1), des masques faciaux (5), des gants et des charlottes pour le personnel impliqué dans des interventions chirurgicales, comprenant un adhésif (7) respectueux de la peau pour fixer hermétiquement des pièces de vêtement sur la peau d'une personne portant le vêtement, **caractérisé en ce que** l'adhésif est constitué d'un élastomère de silicone ou d'un adhésif thermofusible et a un poids par unité de surface de 50 g/m² ou plus et une douceur de 20 à10 mm (mesurée à l'aide d'un procédé basé sur les normes ASTM D 937 et ASTM D 51580); de sorte que plus l'adhésif est doux, plus le poids par unité de surface requis pour la fermeture hermétique soit faible.

2. Vêtement selon la revendication 1, **caractérisé en ce que** l'adhésif(7) est étanche aux fuites selon le test de fuites MHC avec une profondeur de rainure de 50 micromètres.

3. Vêtement selon la revendication 2, **caractérisé en ce que** l'adhésif (7) est étanche aux fuites selon le test de fuites MHC avec une profondeur de rainure de 75 micromètres.

4. Vêtement selon la revendication 3, **caractérisé en ce que** l'adhésif (7) est étanche aux fuites selon le test de fuites MHC avec une profondeur de rainure de 150 micromètres.

5. Vêtement selon la revendication 4, **caractérisé en ce que** l'adhésif (7) est étanche aux fuites selon le test de fuites MHC avec une profondeur de rainure de 200 micromètres.

6. Vêtement selon une ou plusieurs des revendications 1 à 5, **caractérisé en ce que** l'adhésif (7) a un poids par unité de surface de 80 g/m² ou plus et une douceur de plus de 10 mm (mesurée à l'aide d'un procédé basé sur les normes ASTM D 937 et ASTM D 51580).

7. Vêtement selon une ou plusieurs des revendications 1 à 5, **caractérisé en ce que** l'adhésif (7) a un poids par unité de surface de 200 g/m² ou plus et une douceur de plus de 10 mm (mesurée à l'aide d'un procédé basé sur les normes ASTM D 937 et ASTM D 51580).

8. Vêtement selon une ou plusieurs des revendications 1 à 6, **caractérisé en ce que** l'adhésif (7) a une douceur de plus de 12 mm (mesurée à l'aide d'un procédé basé sur les normes ASTM D 937 et ASTM D 51580).

9. Vêtement selon la revendication 8, **caractérisé en ce que** l'adhésif (7) a une douceur de plus de 14 mm (mesurée à l'aide d'un procédé basé sur les normes ASTM D 937 et ASTM D 51580).

10. Utilisation d'un adhésif (7) respectueux de la peau constitué d'un élastomère de silicone ou d'un adhésif thermofusible et ayant un poids par unité de surface de 50 g/m² ou plus et une douceur de 20 à 10 mm (mesurée à l'aide d'un procédé basé sur les normes ASTM D 937 et ASTM D 51580), en sorte que plus l'adhésif est doux, plus le poids par unité de surface requis pour la fermeture hermétique soit faible, pour la fixation amovible et rattachable d'un masque facial (5) à un utilisateur.

11. Utilisation d'un adhésif respectueux de la peau constitué d'un élastomère de silicone ou d'un adhésif thermofusible et ayant un poids par unité de surface de 50 g/m² ou plus et une douceur de 20 à 10 mm (mesurée à l'aide d'un procédé basé sur les normes ASTM D 937 et ASTM D 51580), en sorte que plus l'adhésif est doux, plus le poids unité de surface requis pour la fermeture hermétique soit faible, pour la fixation d'une charlotte chirurgicale à un utilisateur de sorte qu'elle soit scellée sur la peau et rattachable.

12. Utilisation d'un adhésif respectueux de la peau constitué d'un élastomère de silicone ou d'un adhésif thermofusible et ayant un poids par unité de surface de 50 g/m² ou plus et une douceur de 20 à 10 mm(mesurée à l'aide d'un procédé basé sur les normes ASTM D 937 et ASTM D 51580), en sorte que plus l'adhésif est doux, plus le poids par unité de surface requis pour la fermeture hermétique soit faible, pour la fixation de l'encolure (2) et/ou des extrémités des manches (3,4) d'une blouse chirurgicale à la peau d'un utilisateur de manière étanche et refermable.

13. Utilisation d'un adhésif respectueux de la peau constitué d'un élastomère de silicone ou d'un adhésif thermofusible et ayant un poids par unité de surface de 50 g/m² ou plus et une douceur de 20 à 10 mm (mesurée à l'aide d'un procédé basé sur les normes ASTM D 937 et ASTM D 51580), en sorte que plus l'adhésif est doux, plus le poids par unité de surface requis pour la fermeture hermétique soit faible, pour la fixation d'une partie d'un gant chirurgical qui s'étend autour de l'avant-bras d'un utilisateur, à la peau d'un utilisateur de manière étanche et refermable.
